# EUROPEAN PATENT SPECIFICATION

(11) **EP 1 006 959 B1**
(45) Date of publication and mention of the grant of the patent: **09.04.2003**
(21) Application number: 95923623.3
(22) Date of filing: 17.05.1995
(51) Int. Cl.: A61F 4/00, G09B 21/00, A61F 9/08, A61F 11/04

(54) **A PORTABLE MODULAR ALARM SYSTEM**
TRAGBARES MODULARES ALARMSYSTEM
SYSTEME D'ALARME MODULAIRE ET PORTATIF

(43) Date of publication of application: 14.06.2000
(73) Proprietor: GN Transistor Aktiebolag, 113 49 Stockholm (SE)
(72) Inventor: AHGREN, Ragnar, S-741 31 Knivsta (SE)
(74) Representative: Örtenblad, Bertil Tore
(86) International application number: SE9500551
(87) International publication number: WO96036301

(56) References cited:
- EP-A- 0 326 129
- GB-A- 2 138 616
- SE-A- 8 401 387
- SE-B- 445 607

## Description

### FIELD OF INVENTION

The present invention relates to a portable modular alarm system for persons who have impaired hearing and/or impaired vision and persons who are deaf and deaf-blind, wherein the system is designed to recognize acoustic signals in the surroundings and to deliver alarm signals to auxiliary devices, such as to an alarm centre, wherein the system includes a battery-powered wristworn receiver for receiving digital radio signals and a sound recognizing unit which is able to receive and identify acoustic signals, such as telephone signals, doorbell signals, fire-alarm signals or signals from a broadcasting receiver equipped with a radio data system (RDS), and to deliver digital radio signals to the wristworn receiver in response to these acoustic signals, wherein the wristworn receiver includes mechanical time indicating means, optically readable means with symbols corresponding to respective acoustic signals, and means which function to deliver to the wearer vibrotactile signals having characteristics which correspond to respective acoustic signals.

### DESCRIPTION OF THE BACKGROUND ART

Persons whose hearing and/or vision is/are impaired will obvious have difficulties in perceiving signals in their surroundings. Such signals may be signals from smoke detectors, general alarm messages broadcasted over a public radio system with RDS (Radio Data System), telephone signals, doorbell signals or signals deriving from electronic child minding apparatus. The ability to perceive such signals is a necessary condition for a normal life with a normal sense of safety and security. Although systems for transmitting signals from the surroundings are already in existence, these systems are permanent installations. The main drawback with permanent, or static, systems is that they can only be of assistance in those places in which they are installed. Furthermore, a permannet installation is very expensive.

### SUMMARY OF THE INVENTION

The object of the present invention is to provide a system which does not need to be permanently installed but which can be carried easily between different localities and also on journeys.

A portable warning system for the deaf is disclosed in British Patent Application GB 2138616A.

The invention is intended for persons with impaired hearing mainly characterized by a system which includes a portable sound recognizing unit which is constructed to produce digital radio signals in response to acoustic signals, for instance signals deriving from doorbells, telephones, fire alarms, and like devices, wherein the receiver is a wristworn receiver in the form of a wristwatch having mechanical hands and a liquid crystal display divided into a given number of segments and designed to generate in respective segments symbols corresponding to such acoustic signals as telephone signals, doorbell signals, fire alarm signals and signals from corresponding devices, in response to received digital radio signals. The system is also adapted to European standards for emergency telephones, PET (Personal Emergency Telephone), and general alarms broadcasted on general radio networks.

The wristworn receiver has the form of a wristwatch which includes a watch face or dial and mechanical time indicating devices in the form of watch hands, and embodies a digital radio signal receiver and to deliver a characteristic vibrotactile signal to the wearer in response to said signals and to show an associated symbol on the watch dial. Each symbol clearly discloses the type of respective event to which the wearer must be made aware. The nature of the vibrotactile signal can be quickly learned and recognized by the wearer. The wristworn receiver also includes a transmitter which is adapted to activate a PET telephone (Personal Emergency Telephone). The transmitter is activated by holding a button on the wristworn receiver depressed for a given length of time. The transmitter delivers a digital radio signal which identifies the distressed person and the type of assistance required, this information being forwarded to an alarm centre by the PET telephone.

The wristworn receiver, which is preferably worn only during the daytime, includes a battery charging unit which is powered from the mains with the aid of a mains adapter, and includes a built-in rechargeable battery have an effective operating time of about twenty-four hours, for instance. The receiver is preferably plugged into the battery charging unit overnight and connected galvanically thereto. The receiver is able to receive signals as normal while the battery is being recharged. The vibrotactile signals that characterize respective different events are transmitted by the charging unit to a connected external vibrator placed beneath the pillow of the person concerned when sleeping. The battery charging unit also has a built-in microphone with adjustable threshold sensitivity. The microphone picks up acoustic signals which the wristworn receiver attached to the charging unit is unable to perceive and activates the connected external vibrator. The handicapped person will then be made aware of events in his/her surroundings even while sleeping, which greatly enhances the overall safety and security afforded by the system. For instance, the system enables the sleeping person to be awakened by a fire alarm, a telephone or some other warning signal. The battery charging unit includes two switches, one for switching-off the external vibrator and one for setting the face or dial of the wristworn receiver to the correct time at that moment. The battery charging unit also includes a first light-emitting diode which indicates when the wristworn receiver is correctly connected, and a second light-emitting diode which indicates the flow of electric current to the internal battery of the unit. A third light-emitting diode indicates connection of the mains adapter, and a fourth light-emitting diode (red) indicates when the battery is low. This indication will result in activation of the external vibrator.

The digital radio signals received by the wristworn receiver are transmitted from a central sound recognizing unit, hereinafter referred to as the TSD unit (Transett Sound Detector). The TSD unit includes essentially a microphone with amplifier, a signal processing means for real time processing of the acoustic signals registered by the microphone, and a radio transmitter for transmitting digital radio signals. The radio transmitter is modulated with digital signals that represent the acoustic signal recognized by the TSD unit, for instance a doorbell, telephone, fire alarm, etc. The unit can be set to a learning mode with the aid of a keypad, and the characteristics of a specific sound can be stored in the unit through the medium of the keypad. In its operative mode, the unit recognizes the sounds that have been entered and send corresponding digital radio signals to the wristworn receiver. The TSD unit is placed in the room or location occupied by the handicapped person, so that he/she is able to perceive the acoustic signals generated by the unit.

Each wristworn receiver has an individual address (A-address) to which the TSD unit can be adjusted. All wristworn receivers also include a common address code (B-address) for receiving general alarm messages broadcast on general radio networks. The A-address code is entered with the aid of a hexadecimal switch in th TSD-unit and the B-address code for the RDS-alarm is permanently incorporated in the same unit. When an alarm signal is sent from the wristworn receiver to the PET telephone, there is used a telephone adapted address code (C-address).

Child minding equipment included in the system comprises a microphone-amplifier combination capable of activating a radio transmitter. When the microphone picks up sound which lies above a certain level and which continues for a given length of time, the transmitter is activated to transmit a digital radio signal to the wristworn receiver. The signal has an A-address specific to the wristworn receiver concerned. The A-address can be entered with the aid of a hexadecimal switch in the child minding equipment. The child minding equipment may be either battery operated or mains operated through a built-in mains adapter.

### BRIEF DESCRIPTION OF THE DRAWINGS

So that the invention will be more readily understood and further features thereof made apparent, the invention will now be described in more detail with reference to the accompanying schematic drawings, in which
Figure 1 is an overview of the inventive system;
Figure 2 is a block schematic illustrating one embodiment of the wristworn receiver;
Figure 3 illustrates symbols that can be generated in the segmented LCD layer;
Figure 4 illustrates one embodiment of the TSD unit in a physical form;
Figure 5 is a principle function diagram; and
Figure 6 is a block schematic illustrating child minding equipment.

### DETAILED DESCRIPTION OF PREFERRED EMBODIMENTS

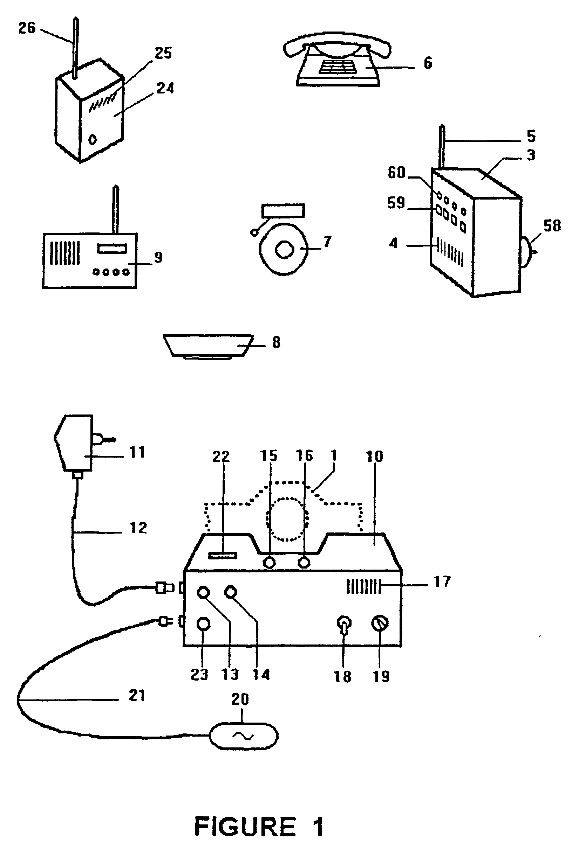

The inventive system illustrated in Figure 1 includes a wristworn receiver 1 which resembles a wristwatch having a round dial or face of conventional size. The system also includes a mains connected TSD unit 3 which incorporates a microphone 4 which picks up acoustic signals radiated to the surroundings. The TSD unit has a built-in radio transmitter and an external transmitter antenna 5. The acoustic signal sources, such as telephone 6 and doorbell 7, are often placed in the proximity of one another, whereas the fire alarm 8 or smoke detector, may be located further away. The system also includes an RDS-receiver 9 which is able to receive general alarm messages broadcast on radio networks for instance, and to deliver acoustic signals. The RDS-receiver may conveniently be battery-operated and is therefore not limited with regard to its location. The TSD-unit is plugged into a wall socket located closer to the telephone 6 and the doorbell 7 than the fire alarm 8, which normally gives out a very loud sound. The illustrated system also includes a battery charging unit 10 into which the wristworn receiver 1 can be plugged, which is preferably done at night. Electric current is supplied to the unit 10 through a mains adapter 11 plugged into a wall socket and connected to the unit 10 by a lead 12 having a standard 12 volt fuse (direct current). The unit 10 has built therein a chargeable battery which when fully chatged will last for about twenty-four hours and which charges when the mains adapter is connected. The unit also includes a light emitting diode 13 which lights up when the battery is being charged, and a light emitting diode 14 which indicates when the battery is low. When the battery runs down to an unhealthy level, an external vibrator 20 connected to the unit 10 will be activated. The unit also includes a further light emitting diode 15 which indicates correct connection of the wristworn receiver 1 in the battery recharging unit 10, and a further light emitting diode 16 which indicates that charging current flows to the battery of the wristworn receiver 1. The unit 10 has a built-in microphone which can be switched on and off by means of a switch 18. The response threshold of the microphone 17 can be adjusted by means of a knob 19. Sound levels above the set threshold level will cause the external vibrator 20 to vibrate, said vibrator being connected to the unit by lead 21. The external vibrator can be switched off, by pressing a button 22 provided on the unit 10 to this end. The unit also includes a press button 23 for setting the time on the dial or face 2 of the wristworn receiver 1.

The inventive system illustrated in Figure 1 also includes a microphone-based child minding device 24. The device is battery driven and includes a built-in microphone 25 which picks up the child's or baby's cries and screams of a given loudness and duration. When preset criteria are fulfilled, the microphone signal is able to activate a built-in radio transmitter which transmits a digital radio signal to the wristworn receiver 1 on an antenna 26, therewith drawing the wearer's attention to the fact that the baby is crying or screaming.

The telephone 6 of the Figure 1 embodiment may be a PET telephone of existing type. The wearer is able to control the wristworn receiver 1 to transmit a digital radio signal, by holding the button 27 pressed for a predetermined length of time. The digital radio signal activates the PET telephone, such as to dial a predetermined emergency number. This procedure is carried out by the wearer in the event of an emergency when immediate assistance is required.

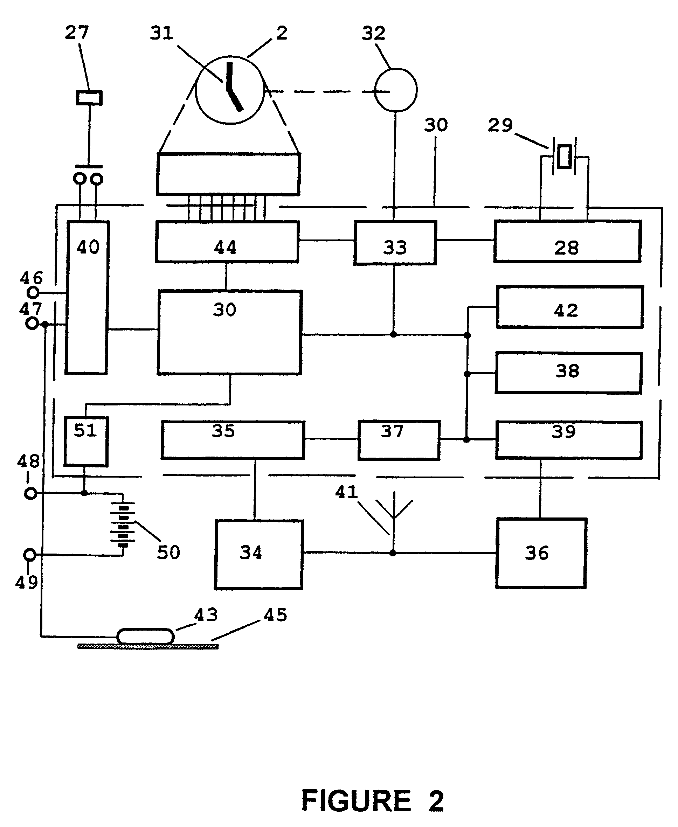

Figure 2 is a block schematic illustrating one embodiment of the wristworn receiver 1. The receiver includes a quartz crystal controlled clock circuit 28 having control crystals 29 (e.g. with a fundamental resonance of 32 MHz) which controls showing of the time and determines the clock frequency of a microprocessor 30, said microprocessor controlling all receiver activities. The LCD-based dial 2 has a layer which includes a plurality of segments for showing a requisite number of symbols and mechanical time indicators 31, i.e. hands. The mechanical hands 31 are driven by a stepping motor 32 which includes a drive circuit 33. Alternatively, the dial 2 may be comprised of two LCD layers, an upper layer which shows the actual time, either by means of hand segments or digitally, and a lower layer showing the symbol that corresponds to a warning signal transmitted from the TDS unit. This latter arrangement obviates the need of the stepping motor 32 and drive circuit 33. Also included is a radio receiver 34 with address and data decoder 35. The wristworn receiver 1 also includes a radio transmitter 36 with an alarm signal output, said transmitter being modulated with digital data from the address memory 37 and a PROM memory 42. Data is transmitted in an appropriate form in the encoding circuit 39, prior to modulating the transmitter 36. Transmission of an alarm signal is initiated by holding the button 27 depressed for a given predetermined length of time. The button 27 is connected to an interface 40 and actuates the microprocessor 30 through said interface, said microprocessor causing an alarm signal adapted to the PET telephone 6 to be transmitted. The antenna 41 is common to both the radio receiver 34 and the radio transmitter 36 and may be embodied in the wrist strap or bracelet. When digital signals are received in the radio receiver 34, the address carried by the signal is decoded in the decoder 35 and compared with the specific A-address or the common RDS alarm address, i.e. the B-address, stored in the address memory 37. If the address is accepted, received data will address the RAM memory 38 and the PROM memory 42, such as to activate the vibrator 43 via the interface 40, and also the LCD dial 2 via the drive circuit 44. The nature of the vibrotactile signals generated by the vibrator 43 and corresponding symbols shown on the dial 2 are determined by the data received. The wristworn receiver includes a bottom plate 45 which is actuated directly by the vibrator 43 and functions to transmit vibrotactile stimuli to the wearer. The tactile and visual stimuli are presented for a predetermined length of time (about twenty seconds). When the wristworn receiver 1 is plugged into the battery-charging unit 10, the receiver will be connected galvanically to said unit in the illustrated case via terminals 46, 47, 48 and 49. The terminal 46 is connected to the microprocessor 30 through the interface 40, wherein the dial 2 can be set to the correct time by pressing the button 23 on the unit 10 and therewith actuate the terminal 46. The terminal 47 delivers characteristic signals to the external vibrator 20 connected to the battery-charging unit 10. The terminals 48 and 49 are operative in connecting the battery 50 to the battery-charging current. As illustrated in Figure 2, the system also includes a monitoring circuit 51 which in the present case is an analog/digital converter and which periodically checks battery voltage in coaction with the microprocessor 30. When the battery is low, there is shown on the dial 2 a battery symbol which indicates that a predetermined limit value has been reached. All functional blocks in the frame 30 are controlled by the microprocessor 30 and may conveniently be incorporated in one and the same integrated circuit.

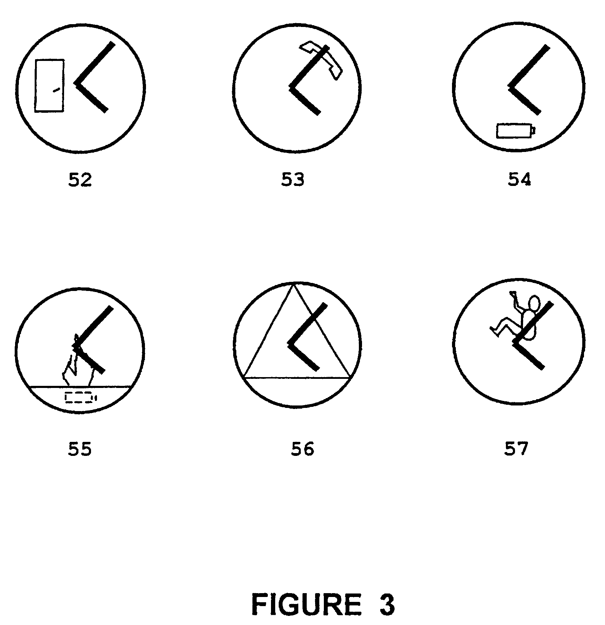

Figure 3 illustrates a few of the symbols that can be created by the segment division in the LCD layer of the dial 2. When all segments are activated, the dial background colour is yellow or some other easily seen colour. When no segment is activated, the dial will appear totally black. As before mentioned, the symbols shown in Figure 3 are among those that may be appropriate to the inventive system. Beginning from the left, the symbols in sequence denote a doorbell 52, a telephone 53, low battery voltage 54, fire alarm 55, a general alarm broadcasted on a radio network 56, and a baby alarm 57. The low voltage symbol can be combined with any of the other symbols. In the case of a fire alarm symbol, the battery symbol will appear black on a yellow background. It will be understood that other combinations are possible within the scope of the invention.

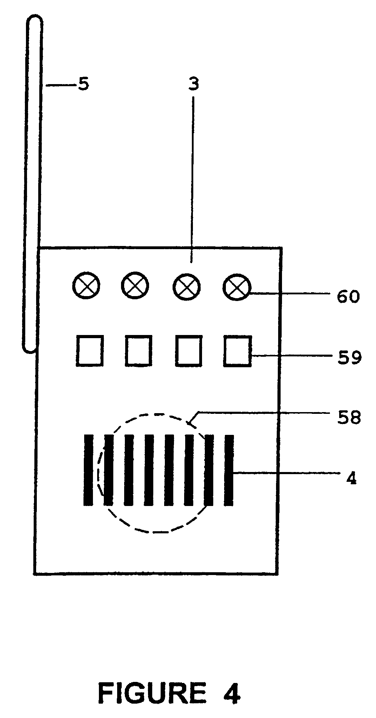

Figure 4 illustrates a physical embodiment of the TSD unit 3. The unit is enclosed in a relatively small rectangular box provided with a mains plug 58 on the rear side thereof and a short antenna 5 on one side. Provided on the front side of the box is a microphone opening 4, and in the illustrated case four push buttons 59 with associated displays in the form of four light emitting diodes 60. The user can set the unit to a learning mode with the aid of the push buttons 59. Thus, four different sounds can be entered in the illustrated embodiment. Correctly stored sound is indicated by a green light on the LED 60 when its associated button is pushed. The learning mode can be repeated subsequent to unplugging the unit. Subsequent to successfully conditioning the unit with respect to said sounds, the unit is plugged into the mains socket and can then recognize and distinguish beteen four different sounds. Recognized sound is indicated with a green light on the light emitting diode adjacent the button used to program or condition said unit. At the same time, a digital radio signal of given duration is sent to the wristworn receiver 1, via the antenna 5. The digital radio signal carries the address of the wristworn receiver 1 and data indicative of the type of acoustic signal concerned.

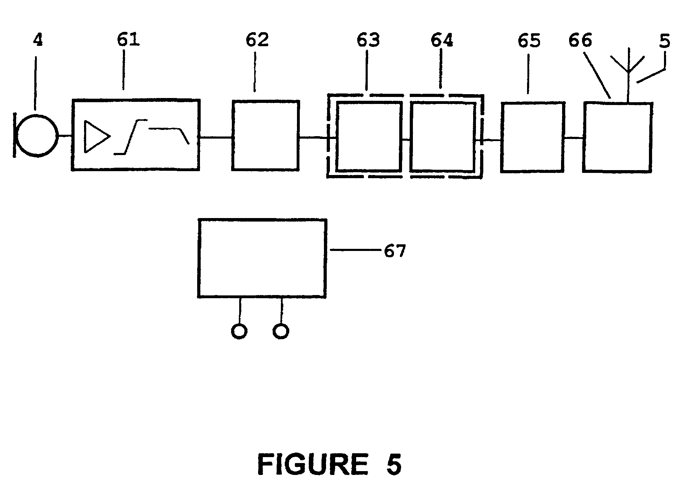

The sound recognizing TSD unit 3 is based on continuous frequency analysis with the aid of known FFT technology (Fast Fourier Transform). The functional block schematic shown in Figure 5 illustrates an example of the procedure followed. The function blocks are all based on well-known techniques. The microphone 4 picks up sound and the microphone signal is amplified, limited in level and in frequency to a frequency of about 8000 Hz in block 61. Block 61 is followed by block 62 which represents an analog-digital converter having, in the present case, a dynamic range of 60 dB and a sampling frequency adapted to the signal bandwidth of 8000 Hz. The digitalized signal is fed serially into a real time signal processor containing function blocks 63 and 64. The FFT analyses are carried out in block 63, within a framework of 256 amplitude levels, after they have been processed with a Hamming-type cosine function to suppress sidebands. The FFT analysis generates 128 positive frequency components (real and imaginary) with a resolution of 64 Hz. This frequency spectrum is compressed, in order to reduce the need of memory and processing capacity. The compression is accomplished by virtue of maintaining said resolution up to 8000 Hz, but is then reduced towards higher frequencies. the compressed spectrum will provide 32 frequency components, which are then put into a logarithmic scale in order to emphasize the weak components. The result is processed to remove components having the lowest amplitude and to provide a positive result. Because the FFT analyses are made on a disturbed and noisy signal, a plurality of FFT spectra are integrated. For instance, if 16 frames each with 256 amplitude values are chosen, there will be obtained an integration time of 256 ms, which may be appropriate with regard to the duration of the acoustic signals.

The 32 frequency components are fed into a neural network (NN) 64 of the Back propagation type. The neural network 64 has the ability to build-up characteristic detectors during the so-called learning mode and store these characteristics for sound recognition, said sound often being mixed with noise. The neural network 64 has three layers when including the input layer. The input layer can handle a vector having 32 components, corresponding to the compressed frequency spectra, while the output can handle a vector having four components which represent the binary code of the signal to be recognized. All inputs are connected to all neurons in the "hidden" layer (eight in number). Each connection includes a weight which determines the degree of coupling between the layers. Two neurons in the "hidden" layer belong to each signal and each neuron in the output layer has two weights. In the learning mode (when the unit is removed from the mains socket), the learning procedure is initiated by pressing a specific button 59, and when the compressed and integrated signal spectra has passed its amplitude maximum. This maximum is the sum of the maximum amplitudes of all frequency components. The frequency components are normalized during the learning mode, wherein the connection weights are determined and stored in the neural network.

In the operational mode (when the TSD unit is plugged into the mains), an FFT analysis is carried out continuously on incoming signals, as represented by function block 63. The signals may be a mixture of noise and signals of relevant interest. The noise recognition process is preferably effected with the same number of compressed and integrated FFT spectra as that used in the learning process, for instance sixteen spectra. The recognition spectra are thus taken from a window with 16 frames of amplitude values (256) which move in time. Recognition tests are run after each new frame, i.e. each sixteenth millisecond. The analyses are made in the same way as in the learning process by normalizing the spectral components, wherein the weights in the connections of the neural network are tabled and compared with the weights stored in the learning process. Significant agreement is obtained with successful recognition, and the light-emitting diode for this specific signal is activated and therewith also the memory circuit 65. The memory circuit 65 contains the specific address for the wristworn receiver (the A-address) and also the common RDS alarm address (the B-address) together with data that represents the specific sound. This data is modulated on the radio transmitter 66 and is transmitted to the wristworn receiver 1 via the antenna 5. The receiver 1 then produces a vibrotactile signal corresponding to said sound, wherewith an associated visual symbol is shown on the dial 2. Unsuccessful learning, or programming, can be erased after unplugging the TSD unit 3. It is only possible to program in a new type of sound when the TSD unit 3 is unplugged and powered by the internal battery in the power supply unit 67. This unit controls this procedure. The weight values obtained in the learning process in a non-volatile memory in the analysis part 63/64.

As an alternative to the radio transmitter in the TSD unit 3, there may be used instead a sound generator which is capable of producing, in this case, four different sound characters, each corresponding to a specific acoustic alarm signal picked up by the microphone 4 and recognized by the TSD unit. Each characteristic sound from the sound generator must have a frequency spectrum that can be readily perceived by the person whose hearing is impaired. Persons who are completely deaf cannot use this embodiment, although it provides an excellent aid to persons whose hearing is moderately impaired.

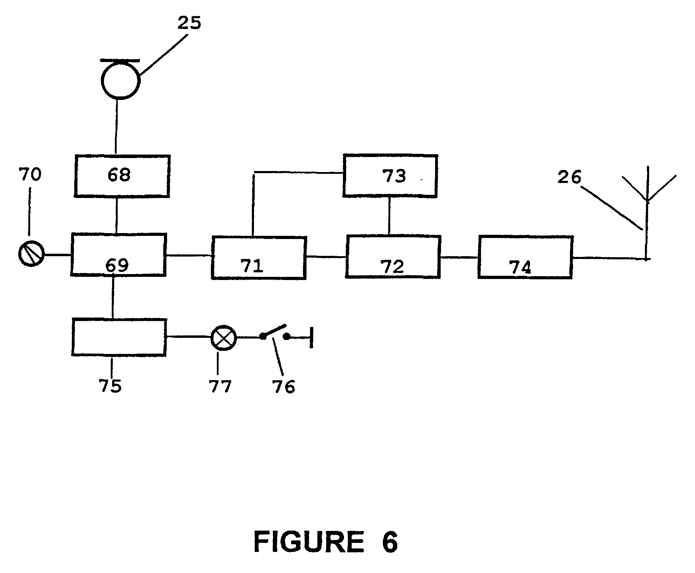

The child minding equipment 24 shown in Figure 1 will now be described with reference to Figure 6. The microphone 25 picks up the child's cries and the microphone signal is amplified in an amplifier 68 whose frequency characteristic is adapted to the frequency spectra of a baby's or child's cries (mean value). When the strength and duration of the signal exceed a predetermined threshold in a threshold circuit 69, a control circuit 71 is activated. The threshold can be preset by a control knob 70. The control circuit 71 in turn activates the decoder 72 and the radio transmitter 74. The decoder 72 obtains address and data from the memory circuit 73. The digital signal modulates the radio transmitter 74 and the radio signal is sent to the wristworn receiver 1 via the antenna 26. The correct address (A-address) in the memory circuit 73 can be set with the aid of an hexadecimal switch. The radio signal received by the wristworn receiver results in a vibrotactile signal characteristic to the child minding alarm, with associated symbols visualized on the dial 2. The baby alarm 24 is powered by a built-in battery 75, which can be switched on and off by the switch 76. Power-on is indicated by the light-emitting diode 77.

The described alarm and warning system has the advantage of not requiring permanent installations, of being easy to handle and adapted for coaction with general alarm systems that exist in Sweden and other countries. The system provides a much safer and more dignified environment to elderly people suffering a hearing and/or sight impairment.

## Claims

1. A system for persons with impaired hearing and/or vision and functioning to recognize acoustic signals in the surroundings and to transmit alarm signals to auxiliary services such as an alarm centre for instance, wherein the system includes
- a portable sound recognizing unit (3) constructed to receive acoustic signals, such as telephone signals, doorbell signals, fire-alarm signals or signals from a radio receiver with radio data system equipment,
and to deliver digital radio signals to a wristworn receiver (1) in response to these acoustic signals
and
- a battery-powered wristworn receiver (1) for receiving digital radio signals, wherein the wristworn receiver (1) has the form of a wristwatch with mechanical time indicating means in the form of mechanical hands (31) and a dial (2) as optically readable means, the dial including a liquid crystal display, LCD, divided into a plurality of segments sufficient to generate telephone, doorbell, fire alarm, symbols, etc., said symbols (52, 53, 54, 55, 56, 57) being generated in response to the aforesaid received digital radio signals,
and includes means which function to deliver to the wearer vibrotactile signals having characteristics corresponding to said acoustic signals.

2. A system according to Claim 1, **characterized in that** the wristworn receiver (1) includes a radio receiver (34) for receiving digital radio signals, a signal decoder (35), an address memory (37) in which there is stored a first address (B) which is common to all wristworn receivers for receiving alarm messages broadcast on radio networks, and a second address (A) specific to respective wristworn receivers for receiving other types of messages, wherein the address delivered by the decoder (35) is compared with addresses (A, B) stored in said memory, wherein when an accepted address is received, the received data is stored in a first memory unit (38), RAM, included in a microprocessor (30) provided in the wristworn receiver, wherein with the aid of this data said microprocessor collects necessary information from a second memory unit (42), PROM, and activates said visual and vibrotactile devices in accordance with said data.

3. A system according to Claim 2, **characterized in that** the wristworn receiver (1) includes a radio transmitter (36) which is activated by a push button (27) on the wristworn receiver, wherein the transmitter becomes active when a given length of time has lapsed after pressing said button and is modulated with digital data which includes an address (C) from the address memory (37) adapted for telephones capable of transferring emergency signals to an alarm centre, and also transmitter identification data from the second memory unit (42), PROM, said data being coded in a coding circuit (39) prior to being delivered to the radio transmitter (36).

4. A system according to Claim 1, **characterized in that** the system includes means (10) for charging a battery in the wristworn receiver (1), wherein the receiver (1) is preferably connected galvanically to said battery-charging means during the night, said means including a chargeable backup battery and means for setting the dial (2) of the wristworn receiver (1) to the correct time.

5. A system according to Claim 4, **characterized in that** the battery-charging means (10) includes an external vibrator (20) which is intended to be placed beneath the pillow of the person concerned and to deliver a vibrotactile signal of the same nature as the vibrotactile device (43) of the wristworn receiver (1) when the wristworn receiver is connected to the battery-charging means (10), wherein the unit (10) has built thereinto a microphone (17) which functions to pick up sounds that exceed a predetermined level and warn the sleeping person of the acoustic alarm through the medium of the vibratory device.

6. A system according to Claim 1, **characterized in that** the sound recognizing unit (3) includes a microphone (4) and a microphone amplifier (61) having a given limited frequency range and automatic level control, an analog-digital converter (62) connected to the amplifier (61) and followed by a real time signal processor (63) which generates with the aid of fast Fourier transform technique a compressed spectrum of the acoustic signals picked up by the microphone (4), which spectrum is integrated over a given time period and fed into a neural network (64) of back propagation kind in a manner such that in the learning mode characteristic values of each particular sound picked up by the microphone are stored and used in the recognition mode of the system, wherein the unit (3) includes four push buttons (59) with associated light-emitting diodes (60) adapted to place the unit (3) in its learning mode and to provide indication of correct learning or recognition of four different sounds, wherein a learning mode can only be achieved when the plug (58) of the unit (3) is disconnected from the mains, in which case the unit (3) is operated by a chargeable battery incorporated in the current supply part (67), wherein the recognition mode is activated when the plug (58) of the unit (3) is connected to mains, wherein successful recognition of sound picked up by the microphone (4) a memory circuit (65) is activated so as to activate a radio transmitter (66) incorporated in the unit (3) and transmit a digital radio signal to the wristworn receiver (1) via an antenna (5), said signal containing the specific A-address settable in the unit (3) and applicable to the wristworn receiver (1) concerned, or the general RDS alarm address, the B-address, and data which discloses the nature of the sound that has been recognized, and wherein said transmission continues for a preset limited time such that the wristworn receiver (1) will present corresponding symbols visually on the dial (2) and the corresponding vibrotactile signal.

## Patentansprüche

1. System für Personen mit geschädigtem Gehör und/oder Sehvermögen, das derart arbeitet, daß es akustische Signale in der Umgebung erkennt und Alarmsignale an Hilfsdienste, wie beispielsweise ein Alarmzentrum, sendet, wobei das System aufweist:
- eine tragbare Geräusche erkennende Einheit (3), die so aufgebaut ist, daß sie akustische Signale empfängt, wie beispielsweise Telefonsignale, Türglockensignale, Feueralarmsignale oder Signale von einem Funkempfänger mit Funkdatensystemeinrichtungen, und daß sie digitale Funksignale an einen am Handgelenk getragenen Empfänger (1) in Reaktion auf diese akustischen Signale übermittelt; und
- einen batteriebetriebenen, am Handgelenk getragenen Empfänger (1) zum Empfangen digitaler Funksignale, wobei der am Handgelenk getragene Empfänger (1) die Form einer Armbanduhr hat, mit mechanischen Zeitanzeigemitteln in der Form mechanischer Zeiger (31) und einem Ziffernblatt (2) als optisch lesbare Mittel,
wobei das Ziffernblatt eine Flüssigkristallanzeige, LCD, aufweist, die in mehrere Segmente unterteilt ist, die ausreichen, um Symbole für Telefon, Türglocke, Feueralarm u.s.w. zu erzeugen, wobei die Symbole (52, 53, 54, 55, 56, 57) in Reaktion auf die zuvor genannten empfangenen digitalen Funksignale erzeugt werden, und wobei Mittel vorgesehen sind, die derart arbeiten, daß sie vibrationstaktile Signale an den Träger abgeben, die den akustischen Signalen entsprechende Charakteristika haben.

2. System nach Anspruch 1, **dadurch gekennzeichnet, daß** der am Handgelenk getragene Empfänger (1) aufweist einen Funkempfänger (34) zum Empfangen digitaler Funksignale, einen Signaldekodierer (35), einen Adressenspeicher (37), in dem eine erste Adresse (B) gespeichert ist, die allen am Handgelenk getragenen Empfängern zum Empfangen vou Alarmmeldungen, die über Funknetze übertragen werden, gemeinsam ist, sowie eine zweite Adresse (A), die den jeweiligen am Handgelenk getragenen Empfängern zum Empfängen anderer Arten von Meldungen spezifisch ist, wobei die von dem Dekodierer (35) abgegebene Adresse mit den in dem Speicher gespeicherten Adressen (A, B) verglichen wird, wobei, wenn eine akzeptierte Adresse empfangen wird, die empfangenen Daten in einer ersten Speichereinheit (38), RAM, gespeichert werden, die in einem Mikroprozessor (30) enthalten ist, der in dem am Handgelenk getragenen Empfänger vorgesehen ist, wobei der Mikroprozessor mit der Hilfe dieser Daten notwendige Informationen von einer zweiten Speichereinheit (42), PROM, sammelt und die visuellen und vibrationstaktilen Einrichtungen in Übereinstimmung mit diesen Daten aktiviert.

3. System nach Anspruch 2, **dadurch gekennzeichnet, daß** der am Handgelenk getragene Empfänger (1) einen Funksender (36) aufweist, der durch einen Druckknopf (27) an dem am Handgelenk getragenen Empfänger aktiviert wird, wobei der Sender aktiv wird, wenn eine bestimmte Zeitdauer nach dem Drücken des Knopfes abgelaufen ist, und mit digitalen Daten moduliert wird, die eine Adresse (C) aus dem Adressenspeicher (37) umfassen, ausgelegt für Telefone, die in der Lage sind, Notrufsignale an ein Alarmzentrum zu übermitteln, sowie außerdem Senderidentifikationsdaten aus der zweiten Speichereinheit (42), PROM, wobei die Daten in einer Kodierschaltung (39) kodiert werden, bevor sie an den Funksender (36) abgegeben werden.

4. System nach Anspruch 1, **dadurch gekennzeichnet, daß** das System Mittel (10) zum Laden einer Batterie in dem am Handgelenk getragenen Empfänger (1) aufweist, wobei der Empfänger (1) vorzugsweise galvanisch während der Nacht mit den Batterielademitteln verbunden ist, wobei die Mittel eine aufladbare Reservebatterie sowie Mittel zum Einstellen des Ziffernblatts (2) des am Handgelenk getragenen Empfängers (1) auf die richtige Zeit aufweisen.

5. System nach Anspruch 4, **dadurch gekennzeichnet, daß** die Batterielademittel (10) einen externen Vibrator (20) aufweisen, der dafür vorgesehen ist, unter dem Kissen der betroffenen Person angeordnet zu werden und ein vibrationstaktiles Signal der gleichen Art wie bei der vibrationstaktilen Einrichtung (43) des am Handgelenk getragenen Empfängers (1) abzugeben, wenn der am Handgelenk getragene Empfänger mit den Batterielademitteln (10) verbunden ist, wobei in die Einheit (10) ein Mikrofon (17) eingebaut ist, das derart arbeitet, daß es Geräusche aufnimmt, die ein vorgegebenes Niveau überschreiten, wobei die schlafende Person durch das Medium der Vibrationseinrichtung über den akustischen Alarm gewarnt wird.

6. System nach Anspruch 1, **dadurch gekennzeichnet, daß** die Geräusche erkennende Einheit (3) aufweist ein Mikrofon (4) und einen Mikrofonverstärker (61) mit einem vorgegebenen begrenzten Frequenzbereich und einer automatischen Niveausteuerung, einen Analog-Digital-Wandler (62), der an den Verstärker (61) angeschlossen ist und dem eine Echtzeit-Signalverarbeitungseinrichtung (63) folgt, die mit der Hilfe einer schnellen Fourier-Transformationstechnik ein komprimiertes Spektrum des von dem Mikrofon (4) aufgenommenen akustischen Signals erzeugt, wobei das Spektrum über eine bestimmte Zeitperiode integriert wird und in ein neuronales Netz (64) der Art der Rückwärtsausbreitung in einer Weise derart eingespeist wird, daß in dem Lernmodus charakteristische Werte jedes bestimmten, von dem Mikrofon aufgenommenen Geräuschs gespeichert werden und in dem Erkennungsmodus des Systems verwendet werden, wobei die Einheit (3) vier Druckknöpfe (59) mit zugeordneten lichtemittierenden Dioden (60) aufweist, die dafür ausgelegt sind, die Einheit (3) in ihren Lernmodus zu bringen und eine Anzeige des korrekten Lernens oder Erkennens von vier verschiedenen Geräuschen zu bilden, wobei ein Lernmodus nur erreicht werden kann, wenn der Stecker (58) der Einheit (3) von der Stromversorgung getrennt ist, wobei die Einheit (3) in diesem Fall durch eine aufladbare Batterie betrieben wird, die in dem Stromversorgungsteil (67) enthalten ist, wobei der Erkennungsmodus aktiviert wird, wenn der Stecker (58) der Einheit (3) mit der Stromversorgung verbunden wird, wobei bei einer erfolgreichen Erkennung eines von dem Mikrofon (4) aufgenommenen Geräuschs eine Speicherschaltung (65) so aktiviert wird, daß ein in der Einheit (3) enthaltener Funksender (66) aktiviert und ein digitales Funksignal über eine Antenne (5) an den am Handgelenk getragenen Empfänger (1) übertragen wird, wobei das Signal die spezifische A-Adresse enthält, die in der Einheit (3) einstellbar und auf den betreffenden, am Handgelenk getragenen Empfänger (1) anwendbar ist, oder die allgemeine RDS-Alarmadresse, die B-Adresse, sowie Daten, die die Art des Geräuschs offenbaren, das erkannt worden ist, und wobei die Übermittlung für eine voreingestellte, begrenzte Zeit derart fortgesetzt wird, daß der am Handgelenk getrage-ne Empfänger (1) entsprechende Symbole visuell auf dem Ziffernblatt (2) und das entsprechende vibrationstaktile Signal darstellt.

## Revendications

1. Système pour des personnes ayant l'audition et/ou la vue affaiblies. ce système servant à reconnaitre des signaux acoustiques dans l'environnement et à transmettre des signaux d'alarme à des services auxiliaires tels que par exemple un centre d'alarme,
**caractérisé en ce qu'**
il comprend :
- un bloc de reconnaissance de sons portatif (3) construit pour recevoir des signaux acoustiques tels que des signaux téléphoniques, des signaux de sonnette, des signaux d'alarme incendie ou des signaux provenant d'un récepteur radio, au moyen d'un équipement de système de données radio, et
délivrer des signaux radio numériques à un récepteur porté au poi gnet ou récepteur bracelet (1), en réponse à ces signaux acoustiques ; et
- un récepteur bracelet alimenté par batterie (1) pour recevoir des signaux radio numériques,
dans lequel
le récepteur bracelet (1) a la forme d'une montre bracelet avec des moyens mécaniques d'indication de l'heure se présentant sous la forme d'aiguilles mécaniques (31) et un cadran (2) servant de moyen de lecture optique, le cadran incluant un afficheur à cristaux liquides (LCD) divisé en un certain nombre de segments suffisants pour générer des symboles de téléphone, de sonnette, d'alarme incendie, etc, ces symboles (52, 53, 54, 55, 56, 57) étant générés en réponse aux signaux radio numériques reçus comme indique ci-dessus, et le récepteur bracelet comprend dcs moyens qui servent à délivrer à celui qui les porte, des signaux vibrotactiles ayant des caractéristiques qui correspondent aux signaux acoustiques.

2. Système selon la revendication 1, **caractérisé en ce que**
le récepteur bracelet (1) comprend un récepteur radio (34) pour recevoir des signaux radio numériques, un décodeur de signaux (35), une mémoire d'adresses (37) dans laquelle sont stockées une première adresse (B) commune à tous les récepteurs bracelets pour recevoir des messages d'alarme diffusés sur des réseaux radio, et une seconde adresse (A) spécifique des récepteurs bracelets respectifs pour recevoir d'autres types de messages, de sorte que l'adresse déliviée par le décodeur (35) est comparée aux adresses (A, B) stockées dans la mémoire lorsqu'une adresse acceptée est reçue, la donnée reçue étant stockée dans un premier bloc de mémoire (38) constitué par une RAM, inclus dans un microprocesseur (30) prévu dans le récepteur bracelet, et
à l'aide de cette donnée, le microprocesseur collecte l'information nécessaire dans un second bloc de mémoire (42) constitué par une PROM, et active les dispositifs visuels et vibrotactiles suivant cette donnée.

3. Système selon la revendication 1,
**caractérisé en ce que**
le récepteur bracelet (1) comprend un émetteur radio (36) activé par un bouton poussoir (27) placé sur le récepteur bracelet, de sorte que l'émetteur devient actif lorsqu'un intervalle de temps donné s'est écoulé après qu'on ait appuyé sur le bouton-poussoir, et l'émetteur est modulé par une donnée numérique comprenant une adresse (c) provenant de la mémoire d'adresse (37) destinée à des téléphones capables de transmettre des signaux d'urgence à un centre d'alarme, ainsi que par une donnéc d'identification d'émetteur provcnant du second bloc de mémoire (49) ou PROM, cette donnée étant codée dans un circuit de codage (39) avant d'être appliquée à l'émetteur radio (36).

4. Système selon la revendication 1,
**caractérisé en ce que**
ce système comprend un chargeur de batterie (10) pour charger une batterie se trouvant dans le récepteur bracelet (1), de sorte que ce récepteur (1) est de préférence connecté galvaniquement au chargeur de hatterie pendant la nuit, ce chargeur comprenant une batterie de secours chargeable ct dcs moycns pour régler lc cadran (2) du récepteur bracelet (1) à l'beure correcte.

5. Système selon la revendication 4,
**caractérisé en ce que**
le chargeur de batterie (10) comprend un vibrateur extérieur (20) destiné à être placé sous l'oreiller de la personne concernée, et à délivrer un signal vibrotactile de même nature que le dispositif vibrotactile (43) du récepteur bracelet (1) lorsque ce récepteur bracelet est connecté au chargeur de batterie (10), ce chargeur (10) comportant un microphone (17) intégré dans celui-ci, ce microphone (17) servant à détecter des sons dépassant un niveau prédéterminé, et à avertir la personne qui dort de l'alarme acoustique, au moyen du dispositif vibratoire.

6. Système selon la revendication 1,
**caractérisé en ce que**
le bloc de reconnaissance de sons (3) comprend un microphone (4) et un amplificateur de microphone (61) ayant une plage de fréquences limitée donnée et une commande de niveau automatique, un convertisseur analogique/numérique (62) connecté à l'amplificateur (61) et suivi par un processeur de signaux en temps réel (63) qui génère, par la technique de transformation de Fourier rapide, un spectre comprimé des signaux acoustiques captés par le microphone (4), ce spectre étant intégré sur une période de temps donnée et envoyé dans un réseau neutre (64) de type à rétro-propagation, de façon que, dans le mode d'apprentissage, des valeurs caractéristiques de chaque son particulier, captées par le microphone, soient stockées et utilisées dans le mode de reconnaissance du système ;
le bloc (3) comprend quatre boutons-poussoirs (59) avec des diodes électroluminescentes associées (60) destinées à placer le bloc (3) dans son mode d'apprentissage et à fournir une indication d'apprentissage ou de reconnaissance correctes de quatre sons différents ;
un mode d'apprentissage ne peut être obtenu que lorsque la prise (58) du bloc (3) est débranchée du secteur, auquel cas le bloc (3) est alimente par une batterie rechargeable incorporée dans la partie d'alimentation de courant (67) ;
le mode de reconnaissance est activé lorsque la prise (58) du bloc (3) est branchée au secteur ;
en cas de reconnaissance couronnée de succès du son capté par le mi crophone (4), un circuit de mémoire (65) est activé de manière à actionner un émetteur radio (66) incorporé dans le bloc (3), et à émettre un signal radio numérique vers le récepteur bracelet (1) par l'intermédiaire d'une antenne (5), ce signal contenant l'adresse A spécifique réglable dans le bloc (3) et applicable au récepteur bracelet (1) concerné, ou l'adresse d'alarme RDS générale, c'est à dire l'adresse B, et une donnée qui décrit la nature du son ayant été reconnu ; et
l'émission se poursuit pendant une période de temps limitée préréglée de façon que le récepteur bracelet (1) fasse apparaître visuellement des symbolcs correspondants sur le cadran (2), ct fournissc lc signal vibrotactile correspondant.
